(19)

Europäisches
Patentamt
European
Patent Office
Office européen
des brevets

(11) **EP 3 348 198 A1**

(12) **EUROPEAN PATENT APPLICATION**
published in accordance with Art. 153(4) EPC

(43) Date of publication:
**18.07.2018 Bulletin 2018/29**

(21) Application number: **16844364.6**

(22) Date of filing: **07.09.2016**

(51) Int Cl.:
*A61B 6/03* (2006.01)      *A61B 5/055* (2006.01)
*G06T 1/00* (2006.01)      *G06T 17/30* (2006.01)

(86) International application number:
**PCT/JP2016/076246**

(87) International publication number:
**WO 2017/043503 (16.03.2017 Gazette 2017/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **11.09.2015 JP 2015179892**

(71) Applicant: **Japan Science and Technology Agency
Kawaguchi-shi, Saitama 332-0012 (JP)**

(72) Inventors:
• **SUZUKI, Munemura**
  **Kagoshima-shi**
  **Kagoshima 891-0117 (JP)**
• **MASUTANI, Yoshitaka**
  **Hiroshima-shi**
  **Hiroshima 731-3194 (JP)**
• **AHARA, Kazushi**
  **Tokyo 164-8525 (JP)**
• **UEDA, Takuya**
  **Chiba-shi**
  **Chiba 260-0842 (JP)**

(74) Representative: **Cabinet Plasseraud
66, rue de la Chaussée d'Antin
75440 Paris Cedex 09 (FR)**

(54) **STRUCTURE ESTIMATION DEVICE, STRUCTURE ESTIMATION METHOD, AND PROGRAM**

(57)     A plurality of points that are present in a target membrane in a medical image are acquired, an initial shape of a polyhedron including the points is then created, normals at the points are then acquired, and a membrane structure is estimated by creating an isosurface using a radial basis function based on the coordinates of and the normals at the points.

FIG.23

EP 3 348 198 A1

## Description

Field

The present invention relates to a structure estimating apparatus, a structure estimating method, and a computer program product.

Background

Having been conventionally disclosed is a technology for generating a simulation model for a surgical operation from a medical image.

Disclosed in a model generating method described in Patent Literature 1 is a technology in which a condition close to an actual surgical operation is reproduced in a simulation by generating an organ model based on geometry information acquired from a medical image, manipulating a template model with a shape of a membrane that is not imaged in a medical image such as a computed tomography (CT) image or a magnetic resonance imaging (MRI) image, and plotting the template model around the organ model.

Citation List

Patent Literature

JP-A-2010-131047

Summary

Technical Problem

The conventional model generating method disclosed in Patent Literature 1, however, has a problem in that the technology merely reproduces a membrane structure around an organ using a template that is generic data, and is not a reproduction of the membrane structure that is unique to the patient.

The present invention is made in consideration of the problem described above, and an object of the present invention is to provide a structure estimating apparatus, a structure estimating method, and a computer program product for visualizing and modelling a membrane structure that has been conventionally impossible to visualize, because, in principle, it is impossible to achieve an appropriate contrast using a medical image unique to a patient.

Solution to Problem

In order to attain this object, a structure estimating apparatus according to one aspect of the present invention is a structure estimating apparatus comprising a point acquiring unit that acquires a plurality of points that are present in a target membrane in a medical image, an initial shape creating unit that creates an initial shape of a polyhedron including the points, a normal acquiring unit that acquires a normal at each of the points, and a membrane structure estimating unit that estimates a membrane structure by creating an isosurface using a radial basis function based on coordinates of and the normals at the points.

The structure estimating apparatus according to another aspect of the present invention is the structure estimating apparatus, wherein the initial shape creating unit creates the initial shape of the polyhedron including the points based on a predetermined number of labels corresponding to coordinates.

The structure estimating apparatus according to still another aspect of the present invention is the structure estimating apparatus, wherein the initial shape creating unit creates the initial shape of the polyhedron including the points based on a relation of connection between the points according to the labels, distances between the points, and a maximum number of edges of the polyhedron having such points as end points.

The structure estimating apparatus according to still another aspect of the present invention is the structure estimating apparatus, wherein the normal acquiring unit selects a reference point from the points, selects a predetermined number of points near the reference point, calculates normals of a predetermined number of triangles formed by the reference point and the points near the reference point, calculates an average normal that is an average of the normals of the predetermined number of triangles, and acquires the average normal as a normal at the reference point.

The structure estimating apparatus according to still another aspect of the present invention is the structure estimating apparatus, wherein the normal acquiring unit also determines a reference normal from normals at points that are assigned with a same label, and corrects an orientation of the normals at the points that are assigned with the same label, based on a direction of the reference normal and adjacency relationships of the points.

The structure estimating apparatus according to still another aspect of the present invention is the structure estimating apparatus, wherein the normal acquiring unit also selects a reference point from the points, weighs the normal at each of the points at vertices adjacent to the reference point, based on the distance between the reference point and the adjacent vertex, using a Gaussian function, and performs smoothing of the normal at the reference point based on the weighing.

The structure estimating apparatus according to still another aspect of the present invention is the structure estimating apparatus, wherein the point acquiring unit also acquires a predetermined number of supplementary points between two points that are end points of an edge of the polyhedron in the medical image, and the normal acquiring unit acquires the normal at each of the points based on the positions of the supplementary points.

The structure estimating apparatus according to still another aspect of the present invention is the structure estimating apparatus, wherein the normal acquiring unit acquires the normal at a selected point that is a point selected from the points.

The structure estimating apparatus according to still another aspect of the present invention is the structure estimating apparatus, wherein the normal acquiring unit causes the initial shape to be displayed, prompts a user to select the selected point from the points, and acquires the normal at the selected point.

The structure estimating apparatus according to still another aspect of the present invention is the structure estimating apparatus, wherein the normal acquiring unit acquires the normal at the selected point based on a plane that includes the selected point by which the polyhedron is formed.

The structure estimating apparatus according to still another aspect of the present invention is the structure estimating apparatus, wherein the normal acquiring unit causes the initial shape to be displayed, prompts a user to make any one or both of an addition of points and a deletion from the points, and acquires the normals at each of the points.

The structure estimating apparatus according to still another aspect of the present invention is the structure estimating apparatus, wherein the point acquiring unit acquires the points that are present in the target membrane, for each of a predetermined number of labels, in the medical image.

The structure estimating apparatus according to still another aspect of the present invention is the structure estimating apparatus, wherein the point acquiring unit acquires the points that are present in the target membrane, by extracting points in a structure other than the membrane by region growing in the medical image.

The structure estimating apparatus according to still another aspect of the present invention is the structure estimating apparatus, wherein the point acquiring unit acquires the points by displaying the medical image and prompting a user to enter the points that are present in the target membrane on the medical image.

The structure estimating apparatus according to still another aspect of the present invention is the structure estimating apparatus, wherein the point acquiring unit acquires the points by displaying the medical image, prompting a user to enter a line delineating the target membrane on the medical image, and identifying the points on the line.

The structure estimating apparatus according to still another aspect of the present invention is the structure estimating apparatus, wherein the membrane structure estimating unit estimates the membrane structure by creating the isosurface based on coordinates of and the normals at the points, using an RBF interpolation that is based on the radial basis function.

The structure estimating apparatus according to still another aspect of the present invention is the structure estimating apparatus, wherein the membrane structure estimating unit also causes the membrane structure to be displayed and prompts a user to make any one or both of an addition of points and a deletion from the points.

The structure estimating apparatus according to still another aspect of the present invention is the structure estimating apparatus, wherein the membrane structure estimating unit also causes the membrane structure to be displayed and prompts a user to make any one, some or all of a change of the normals, an addition of normals, and a deletion from the normals.

The structure estimating apparatus according to still another aspect of the present invention is the structure estimating apparatus, further comprising an image reconstructing unit that creates a reconstruction image that is a reconstruction of the medical image, based on the membrane structure estimated by the membrane structure estimating unit, and an image outputting unit that outputs the reconstruction image.

The structure estimating apparatus according to still another aspect of the present invention is the structure estimating apparatus, wherein the image reconstructing unit creates a reconstruction image corresponding to a clipped section of the membrane based on the membrane structure estimated by the membrane structure estimating unit.

The structure estimating apparatus according to still another aspect of the present invention is the structure estimating apparatus, wherein the image reconstructing unit also creates a reconstruction image in which a structure other than the membrane is visualized based on the medical image, by volume rendering.

The structure estimating apparatus according to still another aspect of the present invention is the structure estimating apparatus, wherein the image reconstructing unit also acquires a value indicating a reliability of the membrane structure estimated by the membrane structure estimating unit, and the image outputting unit also causes the value indicating the reliability of the membrane structure to be displayed.

The structure estimating apparatus according to still another aspect of the present invention is the structure estimating apparatus, wherein the medical image is a CT image or an MRI image in which contrast of the membrane structure is not visually recognizable.

A structure estimating method according to still another aspect of the present invention is a structure estimating method executed by a structure estimating apparatus comprising a point acquiring step of acquiring a plurality of points that are present in a target membrane in a medical image, an initial shape creating step of creating an initial shape of a polyhedron including the points, a normal acquiring step of acquiring a normal at each of the points, and a membrane structure estimating step of estimating a membrane structure by creating an isosurface using a radial basis function based on coordinates of and the normals at the points.

A computer program product according to still another aspect of the present invention is a computer program product having a non-transitory tangible computer readable medium including programmed instructions for causing, when executed by a computer, the computer to perform a structure estimating method comprising a point acquiring step of acquiring a plurality of points that are present in a target membrane in a medical image, an initial shape creating step of creating an initial shape of a polyhedron including the points, a normal acquiring step of acquiring a normal at each of the points, and a membrane structure estimating step of estimating a membrane structure by creating an isosurface using a radial basis function based on coordinates of and the normals at the points.

Advantageous Effects of Invention

According to the present invention, a structure can be reconstructed from a medical image, advantageously, even for a structure such as a membrane structure that is difficult to visualize using a mathematical approach, such as geometry, and anatomical principles. Furthermore, according to the present invention, a computer graphics (CG) representation or a three-dimensional (3D) printout of the geometrical structure of a membrane can facilitate a preliminary examination preceding a surgical operation of the structure whose shape is largely dependent on the individuals, advantageously.

Brief Description of Drawings

FIG. 1 is a block diagram illustrating an example of a configuration of the structure estimating apparatus according to an embodiment.
FIG. 2 is a flowchart illustrating an example of a process performed by the structure estimating apparatus according to the embodiment.
FIG. 3 is a schematic illustrating an example of points that are present in a part of mesocolon in the embodiment.
FIG. 4 is a schematic illustrating an example of mesocolon.
FIG. 5 is a schematic illustrating an example of an initial shape according to the embodiment.
FIG. 6 is a schematic illustrating an example of labels according to the embodiment.
FIG. 7 is a schematic illustrating an example of an isosurface according to the embodiment.
FIG. 8 is a schematic illustrating an example of a point group appended with normal directions in the embodiment.
FIG. 9 is a schematic illustrating an example of volume data according to the embodiment.
FIG. 10 is a schematic illustrating an example of volume data according to the embodiment.
FIG. 11 is a schematic illustrating an example of an isosurface according to the embodiment.
FIG. 12 is a schematic illustrating an example of an isosurface according to the embodiment.
FIG. 13 is a flowchart illustrating an example of a process performed by the structure estimating apparatus according to the embodiment.
FIG. 14 is a schematic illustrating an example of labels according to the embodiment.
FIG. 15 is a schematic illustrating an example of conditions for connecting a point group in the embodiment.
FIG. 16 is a schematic illustrating an example of the distances between the points in the embodiment.
FIG. 17 is a schematic illustrating an example of the initial shape according to the embodiment.
FIG. 18 is a schematic illustrating an example of supplementary points in the embodiment.
FIG. 19 is a schematic illustrating an example of a normal acquiring process according to the embodiment.
FIG. 20 is a schematic illustrating an example of normals in the embodiment.
FIG. 21 is a schematic illustrating an example of labels for correcting the normals in the embodiment.
FIG. 22 is a schematic illustrating an example of orientation unification in the embodiment.
FIG. 23 is a schematic illustrating an example of volume data before clipping in the embodiment.
FIG. 24 is a schematic illustrating an example in which the normals at the points of types 1 to 3 are directed to the normals at the points of type 4 in the embodiment.

FIG. 25 is a schematic illustrating an example of volume data that is to be subjected to the clipping according to the embodiment.

FIG. 26 is a schematic illustrating an example of volume data after the clipping according to the embodiment.

Description of Embodiments

A structure estimating apparatus, a structure estimating method, and a computer program according to an embodiment of the present invention will now be explained in detail with reference to some drawings. The embodiment is, however, not intended to limit the scope of the present invention in any way.

Configuration of Structure Estimating Apparatus 100

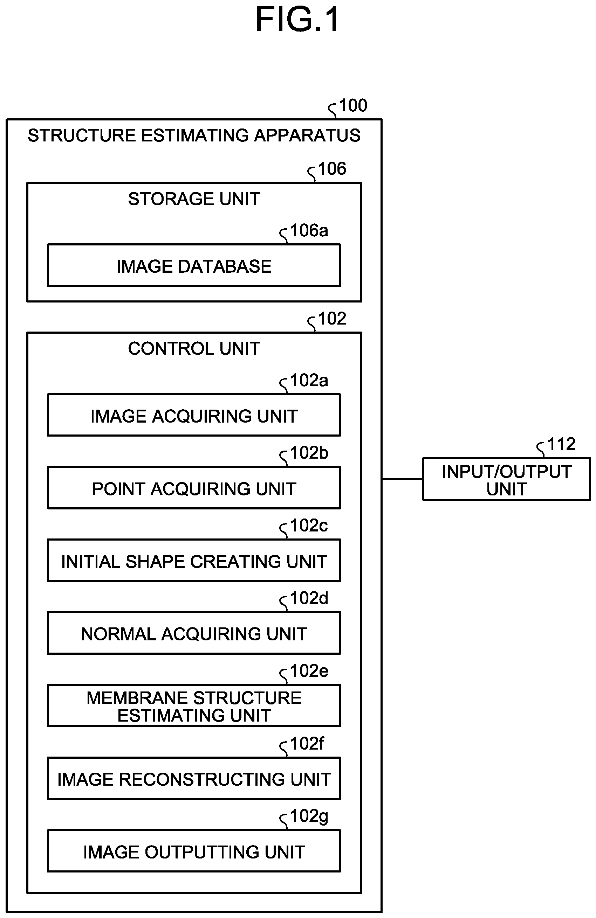

A configuration of a structure estimating apparatus 100 according to an embodiment will now be explained in detail with reference to FIG. 1, and a process and the like according to the embodiment will be explained in detail subsequently. The embodiment described below merely provides an example of the structure estimating apparatus 100 for implementing the technical idea of the present invention, and the technical idea can be applied to any structure estimating apparatus 100 according to other embodiments falling within the scope of the present invention, as defined in the appended claims, in the same manner.

FIG. 1 is a block diagram illustrating an example of a configuration of the structure estimating apparatus 100 according to the embodiment, and illustrating the concept of only the portions that are relevant to the present invention in the configuration.

Explained herein as the structure estimating apparatus 100 according to the embodiment is an apparatus in which all of the elements are housed in one housing, and that executes processes by itself (standalone type), but the structure estimating apparatus 100 may be a conceptual apparatus implemented as separate housings in which the elements are housed, and connected to one another over a network (e.g., cloud computing). In such a configuration, the network has a function for connecting the structure estimating apparatus 100 and external devices, and the like to one another, and may be the Internet, for example.

As illustrated in FIG. 1, the structure estimating apparatus 100 generally includes a control unit 102, a storage unit 106, and an input/output unit 112. The structure estimating apparatus 100 may also include a communication interface unit and an input/output interface unit. These units included in the structure estimating apparatus 100 may be connected to one another communicatively over a communication channel.

The communication interface unit is an antenna that is connected to a communication circuit and/or a telephone circuit, and/or an interface (such as a network interface card (NIC)) that is connected to a communication device such as a router, and may have a function for controlling the communication between the structure estimating apparatus 100 and external devices. The input/output control interface unit is an interface that is connected to the input/output unit 112, and may control the input/output unit 112.

The input/output unit 112 performs input and output (I/O) of data. The input/output unit 112 may be a key input unit, a touch panel, a control pad (such as a touch pad and a game pad), a mouse, a keyboard, and a microphone, for example. The input/output unit 112 may also be a display unit (such as a liquid crystal or organic electroluminescence (EL) display, monitor, and touch panel) for presenting a display screen such as those of an application. The input/output unit 112 may also be an audio output unit (such as a speaker) outputting audio information as sound.

The control unit 102 may control the communication interface unit, the input/output interface unit, and the input/output unit 112.

The storage unit 106 stores therein various types of databases, tables, and/or files (such as an image database 106a). The storage unit 106 may also store therein various application programs (such as a user application).

The storage unit 106 is a storage unit, and may be a memory such as a random-access memory (RAM), a read-only memory (ROM), a fixed disk device such as a hard disk drive, a tangible storage device, such as a solid-state drive (SSD), an embedded multi-media card (eMMC), a flexible disk, and/or an optical disk, or a memory circuit.

Computer programs or the like for performing various processes by giving instructions to a central processing unit (CPU) are stored in the storage unit 106.

Among the elements included in the storage unit 106, an image database 106a stores therein image data related to an image. The image herein may be a medical image. The medical image may be a CT image or an MRI image in which the contrast of a membrane structure is not visually recognizable.

The medical image may be an image in which the contrast of bones, a liver, a kidney, a lung, or blood vessels is visually recognizable. The image data may also be a piece of medical image data for a simulation. The images may also be a reconstruction image.

The control unit 102 is provided as a tangible controller or a control circuit including a CPU, a graphics processing unit (GPU), a digital signal processor (DSP), a large-scale integration (LSI), an application specific integrated circuit (ASIC), and/or a field-programmable gate array (FPGA), for controlling the structure estimating apparatus 100 comprehensively.

The control unit 102 has an internal memory for storing therein control programs, computer programs specifying various processing procedures or the like, and necessary data, and performs information processes for executing various processes based on the computer programs.

The control unit 102 generally includes an image acquiring unit 102a, a point acquiring unit 102b, an initial shape creating unit 102c, a normal acquiring unit 102d, a membrane structure estimating unit 102e, an image reconstructing unit 102f, and an image outputting unit 102g.

Among these units, the image acquiring unit 102a is an image acquiring unit that acquires an image. The image acquiring unit 102a may read image data, and acquire an image based on the image data. The image acquiring unit 102a may acquire the image data from the image database 106a. The image acquiring unit 102a may store the image data in the image database 106a.

The point acquiring unit 102b is a point acquiring unit for acquiring a plurality of points that are present in a target membrane in a medical image. The point acquiring unit 102b may acquire a plurality of points that are present in the target membrane in the medical image, for each of a predetermined number of labels corresponding to coordinates.

The point acquiring unit 102b may acquire a plurality of points that are present in the target membrane by extracting points in a structure other than the membrane by region growing in the medical image. The target membrane may be a ligament or greater omentum, for example.

The point acquiring unit 102b may acquire a plurality of points by causing the input/output unit 112 to display a medical image, and by prompting a user to enter points that are present in the target membrane on the medical image, via the input/output unit 112.

The point acquiring unit 102b may acquire a plurality of points by causing the input/output unit 112 to display the medical image, prompting a user to input a line delineating the target membrane on the medical image via the input/output unit 112, and identifying points on the line.

The point acquiring unit 102b may acquire a predetermined number of supplementary points between two points that are end points of an edge of a polyhedron in the medical image.

The initial shape creating unit 102c is an initial shape creating unit for creating an initial shape of a polyhedron including the points. The initial shape creating unit 102c may create the initial shape of a polyhedron including the points based on a predetermined number of labels corresponding to coordinates.

The initial shape creating unit 102c may create the initial shape of a polyhedron including the points, based on a relation of connection between the points according to the labels, the distances between the points, and the maximum number of edges of a polyhedron having such points as end points.

The normal acquiring unit 102d is a normal acquiring unit that acquires a normal at a point.

The normal acquiring unit 102d may select a reference point from the points, and a predetermined number of points near the reference point. The normal acquiring unit 102d may then calculate the normals of the predetermined number of triangles formed by the reference point and the nearby points, calculate an average normal that is an average of the normals of the predetermined number of triangles, to acquire the average normal as the normal at the reference point.

The normal acquiring unit 102d may determine a reference normal from the normals at the points assigned with the same label, and correct the orientation of the normals at the points assigned with the same label, based on the orientation of the reference normal, and the adjacency relationships of the points.

The normal acquiring unit 102d may select a reference point from the points, weigh the normal at each of the points at the vertices adjacent to the reference point, based on the distance between the reference point and the adjacent vertex, using a Gaussian function, and perform smoothing of the normal at the reference point based on the weighing.

The normal acquiring unit 102d may acquire the normal at a point based on the positions of supplementary points.

The normal acquiring unit 102d may acquire the normals at selected points that are the points selected from the points. The normal acquiring unit 102d may cause the input/output unit 112 to display the initial shape, prompt a user to select the selected points from a plurality of points via the input/output unit 112, and acquire the normals at the selected points.

The normal acquiring unit 102d may acquire the normals at the selected points based on a plane of a polyhedron including the selected points. The normal acquiring unit 102d may cause the input/output unit 112 to display the initial shape, prompt a user to confirm the reliability of the initial shape and to make an addition of points and/or a deletion from the points via the input/output unit 112, and then acquire the normals at the points.

The membrane structure estimating unit 102e is a membrane structure estimating unit for estimating the membrane structure by creating an isosurface using a radial basis function based on the coordinates and the normal at the points. The membrane structure estimating unit 102e may estimate the membrane structure by creating an isosurface based on the coordinates of and the normals at the points, using a radial basis function (RBF) interpolation that is based on a radial basis function.

The membrane structure estimating unit 102e may cause the input/output unit 112 to display the membrane structure, and prompt a user to confirm whether the membrane structure does not contradict with the anatomical structure and to make an addition of points and/or a deletion from the points via the input/output unit 112.

The membrane structure estimating unit 102e may cause the input/output unit 112 to display the membrane structure, and cause a user to confirm whether the membrane structure does not contradict with the anatomical structure, and to make a change of the normals, an addition of normals, and/or a deletion of the normals, via the input/output unit 112.

The image reconstructing unit 102f is an image reconstructing unit for creating a reconstruction image that is a reconstruction of a medical image based on the membrane structure estimated by the membrane structure estimating unit 102e. The image reconstructing unit 102f may create a reconstruction image corresponding to a clipped section of the membrane based on the membrane structure estimated by the membrane structure estimating unit 102e.

The image reconstructing unit 102f may create a reconstruction image in which the structures other than the membrane are visualized from the medical image, by volume rendering, for example. The structures other than the membrane may be nearby structures such as intestine, blood vessels, or bones. The image reconstructing unit 102f may acquire a value representing the reliability of the membrane structure estimated by the membrane structure estimating unit 102e.

The image outputting unit 102g is an image outputting unit for outputting a reconstruction image via the input/output unit 112. The image outputting unit 102g may cause the input/output unit 112 to display a value indicating the reliability of the membrane structure.

The explanation of the example of a configuration of the structure estimating apparatus 100 according to the embodiment is now finished.

Process Performed by Structure Estimating Apparatus 100

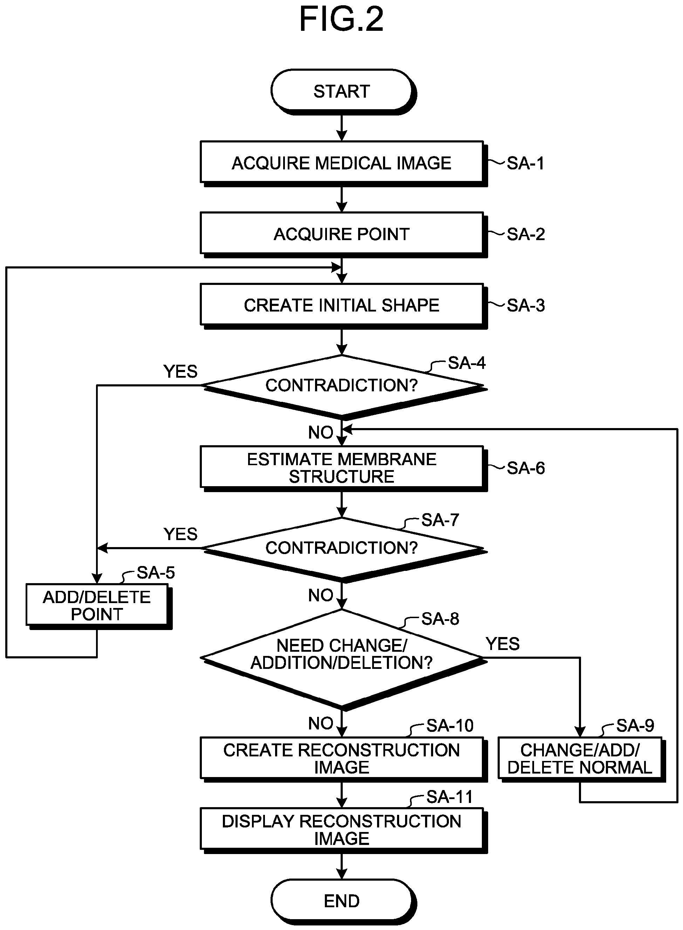

A process performed by the structure estimating apparatus 100 according to the embodiment having the structure described above will now be explained in detail, with reference to FIGS. 2 to 26.

Structure Estimating Process (First Example)

To begin with, an example of a structure estimating process according to the embodiment will now be explained with reference to FIGS. 2 to 12. FIG. 2 is a flowchart illustrating an example of the process performed by the structure estimating apparatus 100 according to the embodiment.

As illustrated in FIG. 2, the image acquiring unit 102a reads a piece of medical image data for a simulation, and acquires a medical image such as a CT image or an MRI image in which the contrast of the membrane structure is not visually recognizable (Step SA-1). The image acquiring unit 102a may read the medical image data from the image database 106a, or read the medical image data from an external device over a network.

The point acquiring unit 102b acquires a plurality of points that are present on the ligament (a point group) in the medical image, for each of a predetermined number of labels corresponding to the coordinates established based on the anatomical principles (Step SA-2). The point acquiring unit 102b may acquire a plurality of points that are present in the ligament in the medical image, by extracting points that are present in the structures other than the membrane by region growing, for each of a predetermined number of labels corresponding to the coordinates established based on the anatomical principles.

The point acquiring unit 102b may acquire a plurality of points by causing the input/output unit 112 to display the medical image and prompting a user (e.g., a user with anatomical knowledge) to enter points that are present in the ligament in the medical image via the input/output unit 112, for each of a predetermined number of labels corresponding to the coordinates established based on the anatomical principles.

The point acquiring unit 102b may acquire a plurality of points by causing the input/output unit 112 to display the medical image, by prompting a user to enter a line delineating the ligament in the medical image via the input/output unit 112, for each of a predetermined number of labels corresponding to the coordinates established based on the anatomical principles, and identifying the points on the line. The point acquiring unit 102b may acquire a plurality of points that are present in the ligament in the medical image by combining these methods.

In other words, in the embodiment, in the process of acquiring a plurality of points (coordinates of a point group) that are classified into a predetermined number of labels (e.g., four labels), a method for extracting points on a blood vessel with region growing, a method for manually entering the coordinates of the points, and/or a method for extracting appropriate points from an input of a line delineating a membrane via a drawing tool may be used.

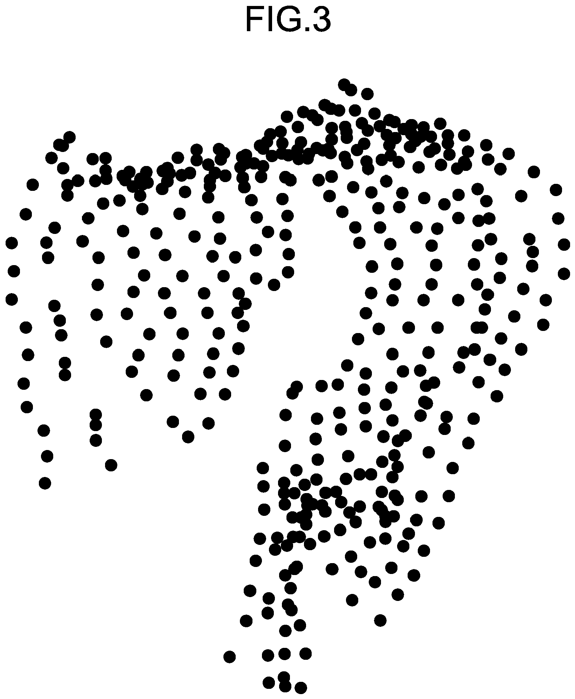

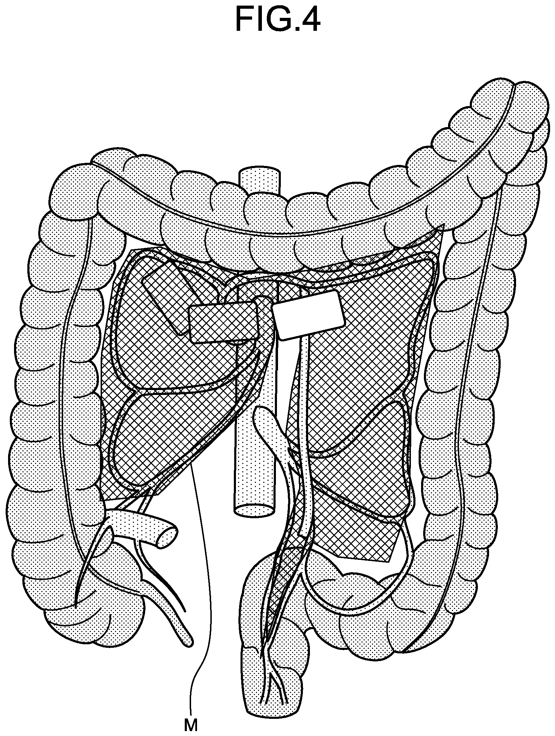

An example of a point input according to the embodiment will now be explained with reference to FIGS. 3 and 4. FIG. 3 is a schematic illustrating an example of the points that are present in a part of ligament (mesocolon) in the embodiment. FIG. 4 is a schematic illustrating an example of mesocolon.

As illustrated in FIG. 3, in the embodiment, when a plurality of points that are present on the mesentery are entered by a user having anatomical knowledge about structures around the colon including the mesocolon M illustrated in FIG. 4 via the input/output unit 112, the points may be acquired as a point group that is present in the mesentery.

In this manner, in the embodiment, a finite number of points that are plotted may be acquired based on the position at which the membrane is presumed to be present, presumed by a user having anatomical knowledge, based on an image that is included in a cross-sectional view of a human body, and in which the membrane is not directly visualized.

Referring back to FIG. 2, the initial shape creating unit 102c creates an initial shape of a polyhedron including the points, based on a predetermined number of labels corresponding to the coordinates, and the normal acquiring unit 102d acquires the normals at the selected points that are points selected from a plurality of points (Step SA-3).

The initial shape creating unit 102c may create the initial shape of a polyhedron including the point based on a predetermined number of labels corresponding to the coordinates, and the normal acquiring unit 102d may acquire the normals at the selected points, by causing the input/output unit 112 to display the initial shape and prompting a user to select the selected points from a plurality of points via the input/output unit 112.

The initial shape creating unit 102c may create the initial shape of a polyhedron including the point based on a predetermined number of labels corresponding to the coordinates, and the normal acquiring unit 102d may acquire the normals at the selected points based on the planes of the polyhedron including the selected points.

The normal acquiring unit 102d may acquire the normals (estimate the directions of the normals) at a low density in a region where the curvature radius of the initial shape is small, and at a high density in a region where the curvature radius is large.

In other words, in the embodiment, in the process of creating the initial shape of a polyhedron from the acquired coordinates of the point group, and giving normal directions to the point coordinates, it is possible to use a method in which the initial shape is estimated using type of the point group, a method for confirming the reliability of the initial shape, and/or a method for selecting a point group for which the normal directions are to be explicitly indicated, and a point group for which the normal directions are not to be explicitly indicated.

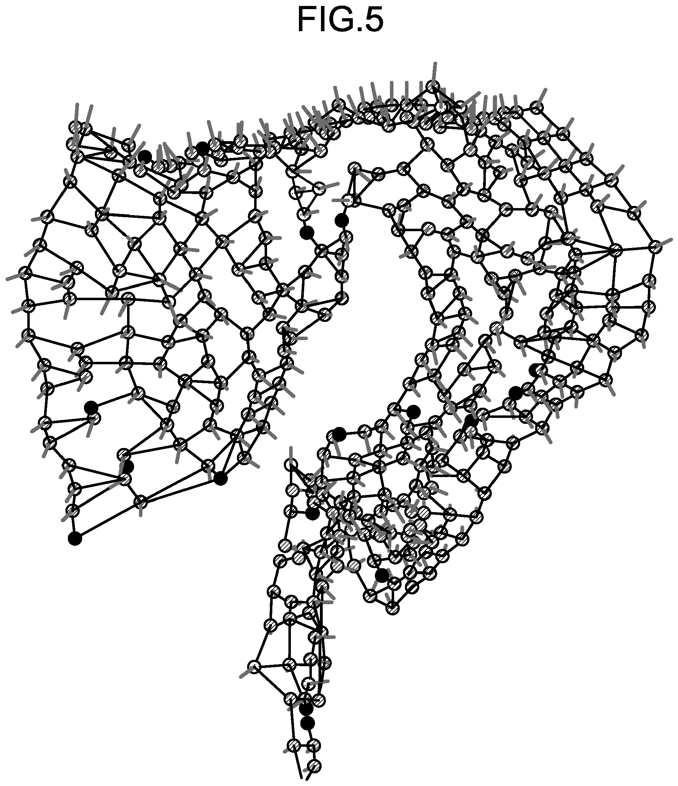

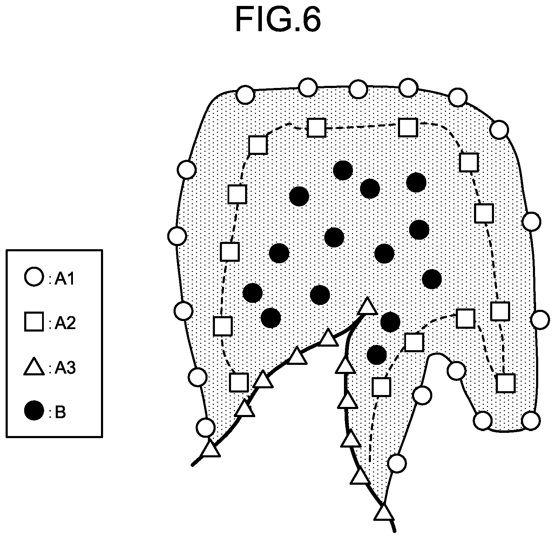

An example of an initial shape creation according to the embodiment will now be explained with reference to FIGS. 5 and 6. FIG. 5 is a schematic illustrating an example of the initial shape according to the embodiment. FIG. 6 is a schematic illustrating an example of labels according to the embodiment.

As illustrated in FIG. 5, in the embodiment, in order to presume the topological shape of the entire membrane from the group of discretely acquired points indicating the position of the mesentery, a relative relation of the acquired point group may be evaluated from the viewpoint of being significantly near, and the initial shape (pseudo graph structure) may be created therefrom.

In the embodiment, for a point group $A=\{a0, a1, a2, ...\}$ and a point group $B=\{b0, b1, b2, ...\}$, the points may be determined to be being significantly nearby defining "a set of oriented edges formed by points that are significantly near to B from A" with the expression SN(A, B), which is explained below.

In other words, in the embodiment, the set of oriented edges is defined by $SN(A, B)=\{(ai, bj)|ai \in A, bj \in B, dist(ai, bj)<dist(ai, B)*c\}$, where dist denotes an Euclidean distance in the space, the constant c is a real number determined by the nature of the point group, and is approximately 1.58, for example, in the mesentery modelling in the embodiment, for example.

As illustrated in FIG. 6, in the initial shape creation (graph structure) according to the embodiment, four types of labels corresponding to the coordinates may be set, including a label A1 for a group of a finite number of points that are located on the center line of the colon, a label A2 for a group of a finite number of points that are located on the outer boundary of the mesentery region (colon-side), a label A3 for a group of finite number of points that are located on the inner boundary of the mesentery region (main-artery-side), and a label B for a group of other points on the mesentery.

In the initial shape (graph structure) creation according to the embodiment, the topological geometrical structure (one-dimensional structure) of the center line of the colon may be identified by establishing a definition $E1=SN(A1, A1)$, and the topological geometrical structure (one-dimensional structure) of the boundary line of the mesentery may be identified by establishing a definition $E2=SN(A2, A2) \cup SN(A3, A3)$.

In the initial shape (graph structure) creation according to the embodiment, the topological geometrical structure (two-dimensional structure) of the plane on which the mesentery and the colon are connected may be identified by establishing a definition $E12=SN(A1, A2) \cup SN(A2, A1)$, and the topological geometrical structure (two-dimensional structure) of the mesentery plane may be identified by establishing a definition $EB=SN(B, B \cup A2 \cup A3)$.

As illustrated in FIG. 5, in the embodiment, the normals at the selected points (normal directions) may be acquired by calculating the vertical vectors of an estimated curved plane.

Referring back to FIG. 2, the initial shape creating unit 102c causes the input/output unit 112 to display the created initial shape, and determines whether there is any contradiction between the initial shape and the anatomical structure by prompting a user to confirm the reliability of the initial shape via the input/output unit 112, that is, to confirm whether there is any contradiction with respect to the anatomical structure, and to input the confirmation result (Step SA-4).

If the initial shape creating unit 102c determines that there is a contradiction at Step SA-4 (Yes at Step SA-4), the process is shifted to Step SA-5.

The initial shape creating unit 102c then prompts a user to make an addition of points and/or deletion from the points that are present in the ligament in the medical image via the input/output unit 112, for each of a predetermined number of labels corresponding to the coordinates established based on the anatomical principles (Step SA-5), and the process is shifted to Step SA-3.

In other words, in the embodiment, a user may be caused to confirm whether the acquired initial shape does not contradict with the anatomical structure, and to make an addition of a point or a deletion of the point.

If the initial shape creating unit 102c determines that there is no contradiction at Step SA-4 (No at Step SA-4), the process is shifted to Step SA-6.

The membrane structure estimating unit 102e estimates the membrane structure by creating an isosurface based on the coordinates of the selected points and the normals at the selected points, using an RBF interpolation that is based on a radial basis function (Step SA-6).

In other words, in the embodiment, in the process of reconstructing volume data from the coordinates of the points group appended with the normal directions (including those without any directions), using an RBF interpolation, a calculation method for RBF interpolation, and/or a method for acquiring the membrane structure using isosurface reconstruction may be used.

For example, in the embodiment, an implicit function s(X) is defined as Equation 1 below, using the coordinates $X=(x, y, z)^T$ of a point that is present in the ligament, a polynomial function $p(X)=c_1+ c_2x+c_3y+c_4z$, a coefficient $\lambda_i (1 \leq i \leq N)$ for the points that are present in the ligament (where N is the number of the points), and a basis function $\phi(r)$.

[Equation 1]

$$s(X) = p(X) + \sum_{i=1}^{N} \lambda_i \phi(|X - X_i|)$$

$$\text{(Where } X \in \boldsymbol{R}^3)$$

$$(1)$$

In the embodiment, $\phi(r)=r$ is then employed in a manner minimizing the energy, and, as an approximating process, $\Lambda=(\lambda_1, ..., \lambda_N)^T$ that are N values of the coefficient $\lambda$, and $c=(c_1, c_2, c_3, c_4)^T$ which is a value of four coefficients are determined.

In the embodiment, volume data is reconstructed by adding a condition defined as following Equation 2 ($P^T\Lambda=0$), as a constraint related to the orthogonality of the points that are present in the ligament, and creating an isosurface in which an implicit function s(X)=s is satisfied, for the scalar value s in X, by solving following Equation 3.

[Equation 2]

$$\sum_{i=1}^{N} \lambda_i = \sum_{i=1}^{N} \lambda_i x_i = \sum_{i=1}^{N} \lambda_i y_i = \sum_{i=1}^{N} \lambda_i z_i = 0 \qquad (2)$$

[Equation 3]

$$\begin{pmatrix} A & P \\ P^T & 0 \end{pmatrix} \begin{pmatrix} \Lambda \\ c \end{pmatrix} = \begin{pmatrix} S \\ 0 \end{pmatrix}$$

$$\left( \text{Where} \quad A_{i,j} = \phi(|X_i - X_j|) = |X_i - X_j|, \ S = (s_1,...,s_N)^T, P = \begin{pmatrix} 1 & x_1 & y_1 & z_1 \\ & \vdots & & \\ 1 & x_N & y_N & z_N \end{pmatrix} \right)$$

$$(3)$$

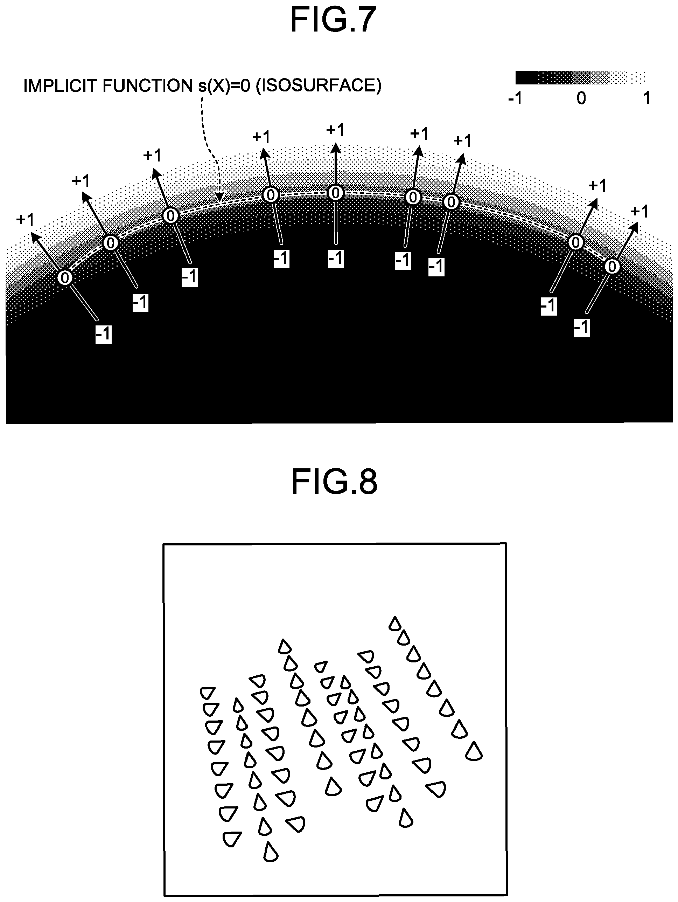

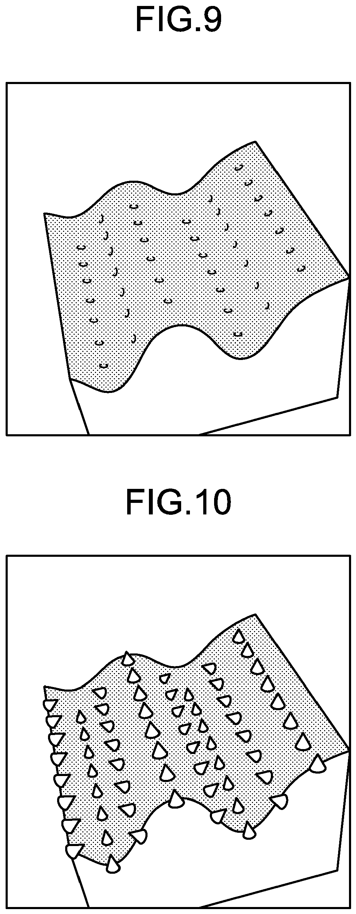

An example of an isosurface creation according to the embodiment will now be explained with reference to FIGS. 7 to 12. FIGS. 7, 11, and 12 are schematics illustrating some examples of an isosurface according to the embodiment. FIG. 8 is a schematic illustrating an example of a point group appended with normal directions in the embodiment. FIGS. 9 and 10 are schematics illustrating examples of volume data according to the embodiment.

As illustrated in FIG. 7, in the embodiment, positions satisfying the implicit function s(X)=0 may be acquired as an isosurface.

In the embodiment, when the coordinates (the positions of cones) and the normal directions (the orientations of the cones) at a plurality of points (point group) that are present in the ligament have been acquired, as illustrated in FIG. 8, the volume data is reconstructed by an RBF interpolation using the implicit function s(X), as illustrated in FIGS. 9 and 10.

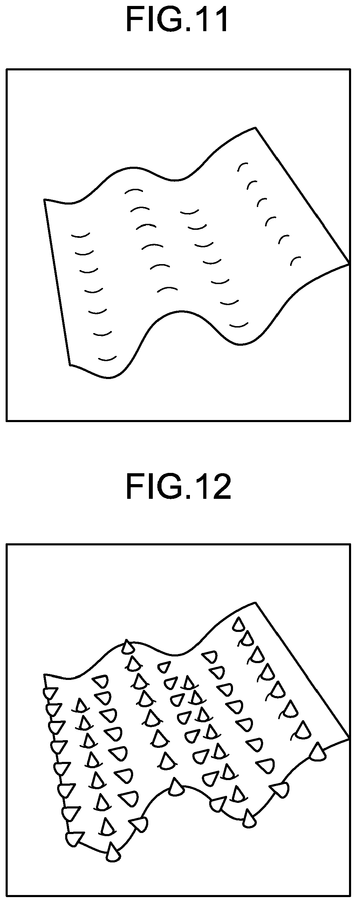

In the embodiment, a plane where the implicit function s(X)=0 is acquired as an isosurface from the reconstructed volume data, as illustrated in FIGS. 11 and 12.

Referring back to FIG. 2, the membrane structure estimating unit 102e causes the input/output unit 112 to display the membrane structure, and determines whether there is any contradiction by prompting a user to confirm whether the membrane structure does not contradict with the anatomical structure, and to enter the confirmation result, via the input/output unit 112 (Step SA-7).

If the membrane structure estimating unit 102e determines that there is a contradiction at Step SA-7 (Yes at Step SA-7), the process is shifted to Step SA-5.

If the membrane structure estimating unit 102e determines that there is no contradiction at Step SA-7 (No at Step SA-7), the process is shifted to Step SA-8.

The membrane structure estimating unit 102e then causes the input/output unit 112 to display the normals, and determines whether any change of the normals at the selected points, any addition of normals to the points other than the selected points, and/or any deletion of the normals at the selected points are required, by prompting a user to confirm the normals at the selected points and to enter the confirmation result via the input/output unit 112, (Step SA-8).

For example, in the embodiment, a user may be prompted to confirm the normals at the selected points via the input/output unit 112 by causing the input/output unit 112 to display cones indicating the positions and the normal directions of a point group that is present in the ligament on the isosurface illustrated in FIG. 12.

In other words, in the embodiment, it is possible to use a method of displaying the normal directions calculated in the initial shape creating process. In the embodiment, it is possible to determine whether any normal direction is to be added by prompting a user to confirm whether the structure acquired by the RBF interpolation does not contradict with the anatomical structure.

If the membrane structure estimating unit 102e determines that any change, addition, and/or deletion is required at Step SA-8 (Yes at Step SA-8), the process is shifted to Step SA-9. In other words, in the embodiment, a user may be prompted to confirm whether the structure acquired from the RBF interpolation does not contradict with the anatomical structure, and to make an addition of a point or a deletion of the point.

The membrane structure estimating unit 102e then prompts a user to change the normals at selected points, to add a normal to a point other than the selected points, and/or to delete the normals from selected points via the input/output unit 112 (Step SA-9), and the process is shifted to Step SA-6.

If the membrane structure estimating unit 102e determines that any change, addition, and/or deletion is not required at Step SA-8 (No at Step SA-8), the process is shifted to Step SA-10.

The image reconstructing unit 102f then creates a reconstruction image that is a reconstruction of the medical image based on the membrane structure estimated by the membrane structure estimating unit 102e, and acquires a value indicating the reliability of the membrane structure (Step SA-10).

The image reconstructing unit 102f may create a reconstruction image corresponding to a clipped section of the membrane based on the membrane structure estimated by the membrane structure estimating unit 102e. The image reconstructing unit 102f may create a reconstruction image in which structures other than the membrane, such as the intestine, blood vessels, or bones, are visualized, by volume rendering, for example. It is also possible for the image reconstructing unit 102f to create a reconstruction image that is a reconstruction of the medical image by combining these methods.

In other words, in the embodiment, in the process of reconstructing the image for a simulation using a curved plane (membrane structure) acquired by an RBF interpolation, it is possible to use a method for clipping the section corresponding to the ligament, a method visualizing the nearby structures such as intestine, blood vessels, and bones, and/or a method for acquiring the reliability of the acquired membrane structure.

The image outputting unit 102g then causes the input/output unit 112 to display the reconstruction image and the value indicating the reliability of the membrane structure, as the data for a simulation (Step SA-11), and the process is ended. The image outputting unit 102g may output a 3D printout of a three-dimensional stereoscopic model of the reconstruction image via the input/output unit 112.

Structure Estimating Process (Second Example)

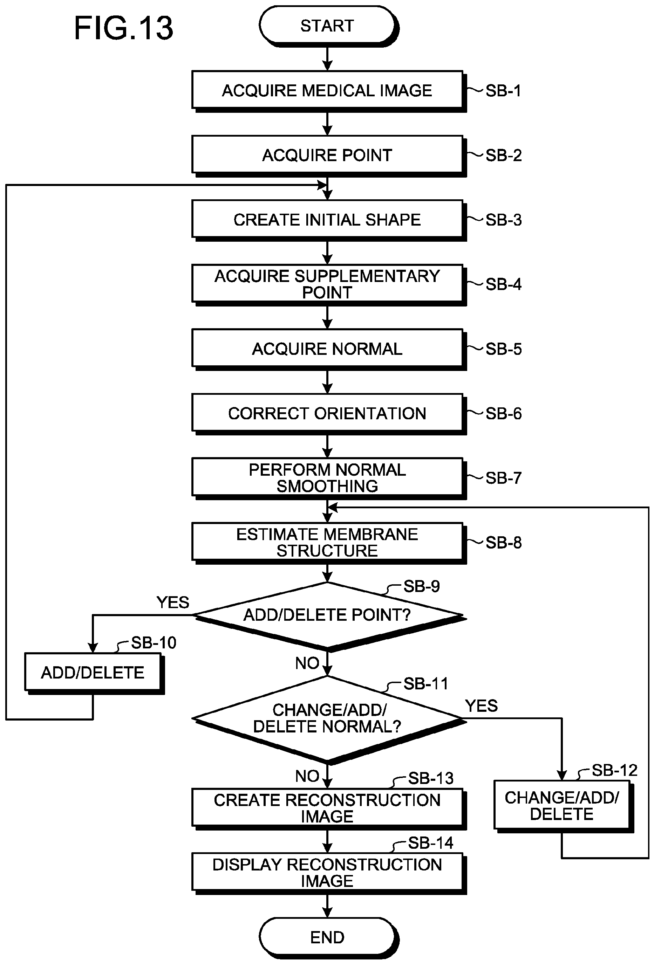

Another example of structure estimating process according to the embodiment will now be explained with reference to FIGS. 13 to 26. FIG. 13 is a flowchart illustrating an example of the process performed by the structure estimating apparatus 100 according to the embodiment.

As illustrated in FIG. 13, the image acquiring unit 102a reads a piece of medical image data, and acquires a medical image such as a CT image or an MRI image in which the contrast of the membrane structure is not visually recognizable (Step SB-1).

When a plurality of points that are present on the ligament (point group) are specified by a user on the medical image, for each of a predetermined number of labels corresponding to the coordinates established based on the anatomical principles, via the input/output unit 112, the point acquiring unit 102b acquires the points (Step SB-2).

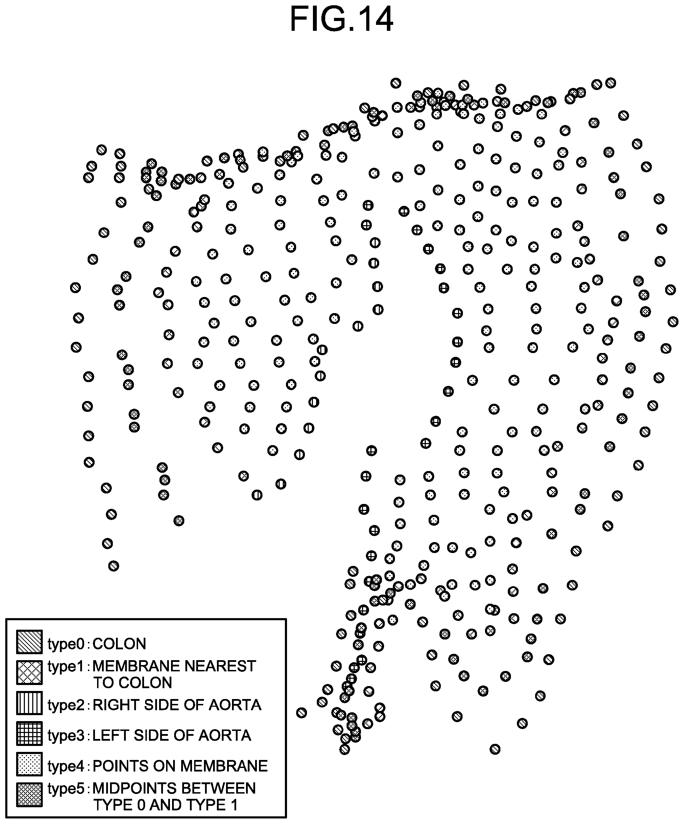

An example of the labeling according to the embodiment will now be explained with reference to FIG. 14. FIG. 14 is a schematic illustrating an example of labels according to the embodiment.

As illustrated in FIG. 14, in the embodiment, points along the colon boundary is labeled as type 0; points along the mesocolon boundary nearest to the colon are labeled as type 1; points along the right side of the main-artery boundary are labeled as type 2; points along the left side of the main-artery boundary are labeled as type 3; points on the mesocolon are labeled as type 4; and midpoints between type 0 and type 1 are labeled as type 5.

A plane reconstructed by the points of type 1, type 2, type 3, and type 4 correspond to the mesocolon, and the section between the points of type 0 and type 1 corresponds to the colon.

In the embodiment, upon creating the initial shape, because there is an unignorable space between type 0 and type 1 representing the boundaries between the colon and the mesocolon, the midpoints between type 0 and type 1 are classified as type 5.

Referring back to FIG. 13, the initial shape creating unit 102c creates an initial shape of a polyhedron including these points, based on the relation of connection between the points according to the labels, the distances between the points, and the maximum number of edges of a polyhedron having such points as end points (Step SB-3).

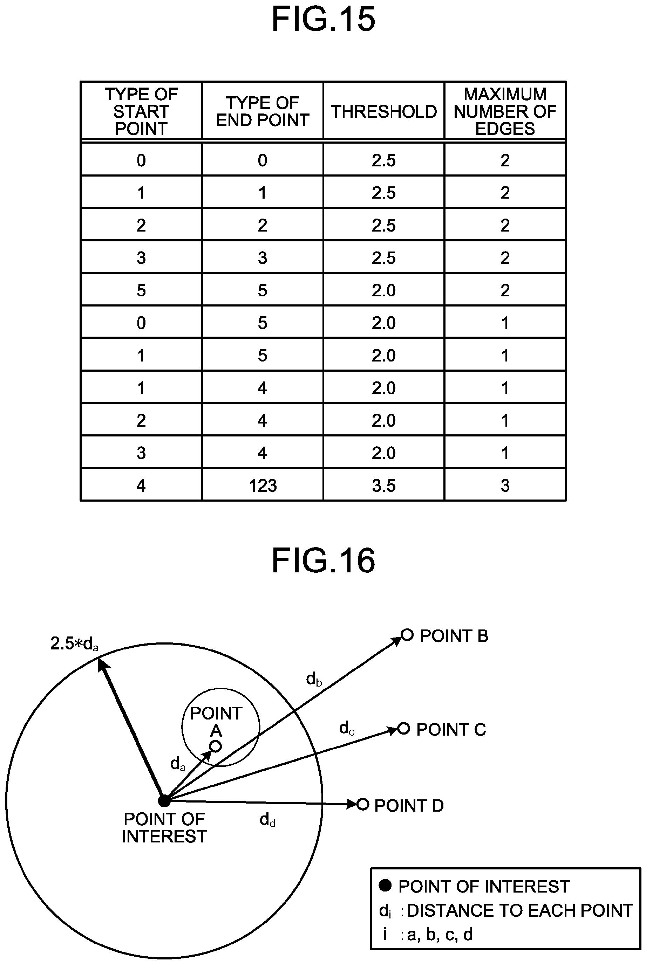

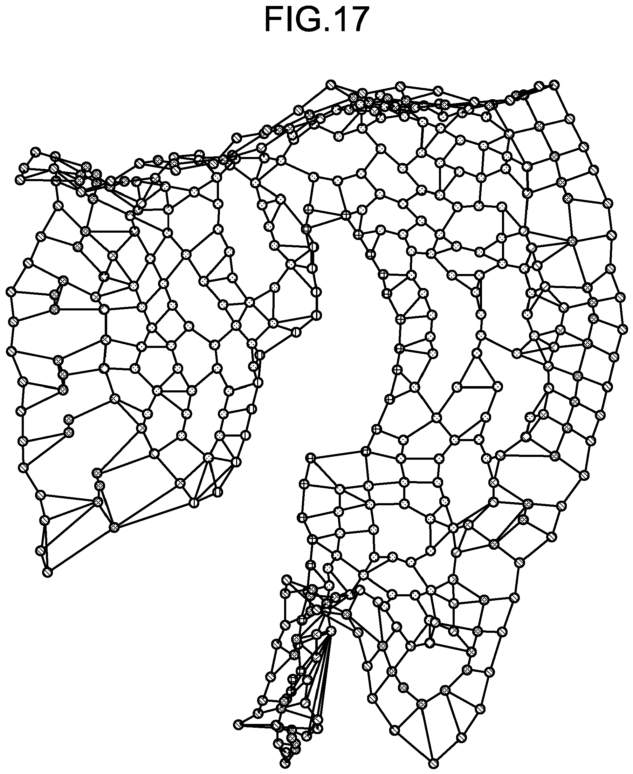

An example of the initial shape creation according to the embodiment will now be explained with reference to FIGS. 15 to 17. FIG. 15 is a schematic illustrating an example of conditions for connecting a point group in the embodiment. FIG. 16 is a schematic illustrating an example of the distances between the points in the embodiment. FIG. 17 is a schematic illustrating an example of the initial shape according to the embodiment.

As illustrated in FIG. 15, in the embodiment, established as conditions for connecting points (nodes) (a start point and an end point) is the number of edges that can be connected at most from a start point having one type (point of interest) to an end point having the same type or a different type and satisfying a predetermined threshold.

As illustrated in FIG. 16, in the embodiment, when points (a start point and an end point) of type 0 are to be connected, a point of interest is always connected to a point A that is at the shortest distance from the point of interest.

As illustrated in FIG. 16, in the embodiment, if there is any point inside of the area of a circle whose radius is equal to the product of the predetermined threshold (2.5) and the distance ($d_a$) of the point A positioned at the shortest distance from the point of interest ($2.5*d_a$), edges are connected to the point of interest, from those at the shortest distance, up to a number specified as the maximum number of edges (two).

In the embodiment, although the maximum number of edges is two, as illustrated in FIG. 15, there are some cases in which only one edge can be connected, as illustrated in FIG. 16.

In the embodiment, the initial shape of a polyhedron (graph structure) is created by connecting the nodes with edges, as illustrated in FIG. 17, based on the conditions illustrated in FIG. 15.

Referring back to FIG. 13, the point acquiring unit 102b then acquires a predetermined number of supplementary points between two points that are the end points of an edge of the polyhedron in the medical image (Step SB-4).

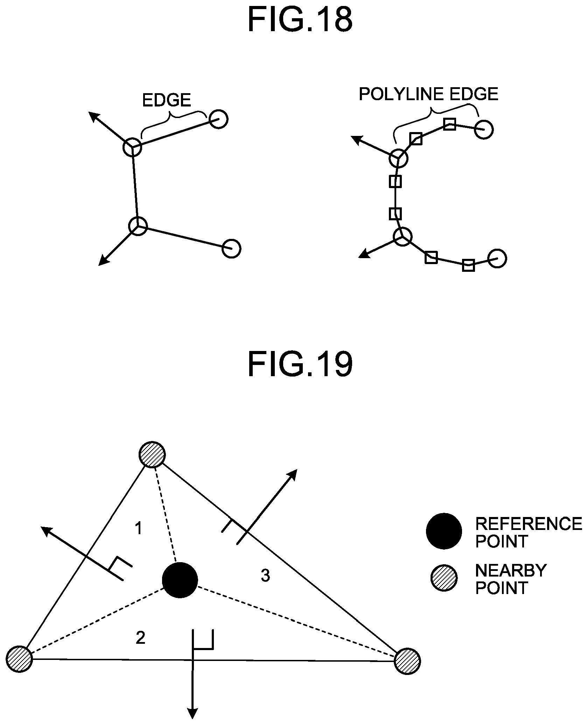

An example of the supplementary points according to the embodiment will now be explained with reference to FIG. 18. FIG. 18 is a schematic illustrating an example of supplementary points in the embodiment.

As illustrated in FIG. 18, in the embodiment, after determining the edge between the nodes that are the elements of the initial shape, two additional supplementary points are added to the edge, to form a polyline edge.

In a normal acquiring process according to the embodiment which is described later, the supplementary points on the edge are slightly moved in such a manner that the edges around the nodes are smoothed out, to make it easy to estimate the vertical direction of the normal.

In the normal acquiring process according to the embodiment which is described later, by making the edge a polyline edge, it becomes easier to estimate whether the vertical directions of the normals at two adjacent nodes extend either on the front side or the reverse side.

Referring back to FIG. 13, the normal acquiring unit 102d selects a reference point from the points, selects a predetermined number of points near the reference point, calculates the normals of a predetermined number of triangles formed by the reference point and the nearby points, and calculates an average normal that is an average of the normals of the predetermined number of triangles based on the positions of the supplementary points, and acquires the average normal as the normal at the reference point (Step SB-5).

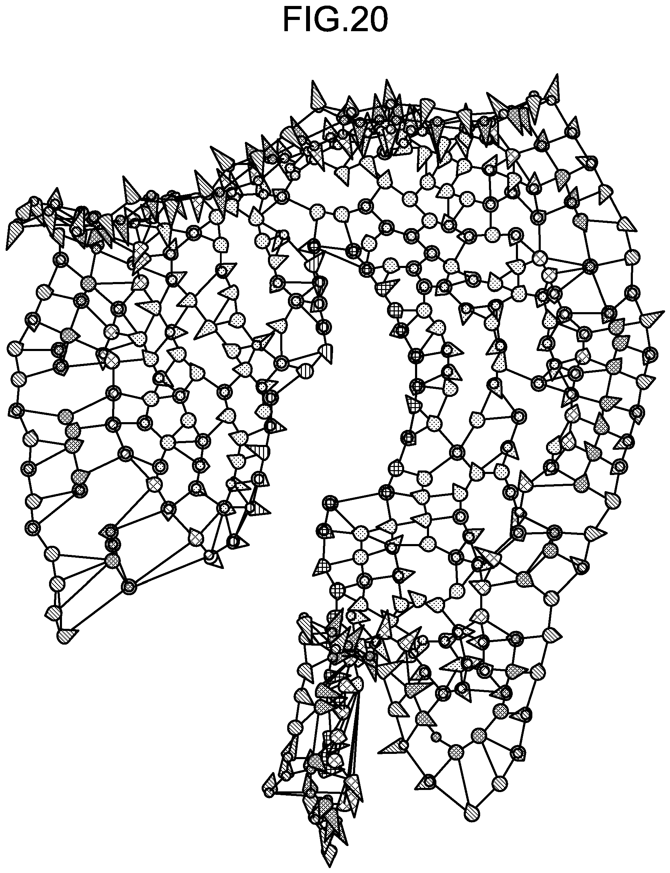

An example of the normal acquiring process according to the embodiment will now be explained with reference to FIGS. 19 and 20. FIG. 19 is a schematic illustrating an example of the normal acquiring process according to the embodiment. FIG. 20 is a schematic illustrating an example of the normals in the embodiment.

As illustrated in FIG. 19, in the embodiment, three points near the reference point are selected, and three triangles formed by the point of interest and the nearby points (triangle 1, triangle 2, and triangle 3) are then formed.

In the embodiment, the normal (normal vector) of each of the three triangles is then calculated, and an average normal that is an average of the three normals is acquired as the normal at the reference point.

In the embodiment, in the process of selecting the three nearby points, if any of the reference points is connected to four or more nearby points via edges, by obtaining every triangle formed by three of the nearby points, the triangles in which the sum of vectors from the reference point to that three nearby points is the smallest may be selected as the triangles for which the normals are to be acquired.

In other words, in the embodiment, by establishing triangles nearest to the reference point as the triangles for which the normals are to be acquired, a more accurate normal can be acquired.

In the embodiment, the normals (cones) of the respective points (nodes) included in the initial shape are acquired, as illustrated in FIG. 20. The direction from the base to the vertex of the cone represents the orientation of the normal.

Referring back to FIG. 13, the normal acquiring unit 102d then determines a reference normal from the normals at the points assigned with the same label, and corrects the orientations of the normals at the points assigned with the same label based on the direction of the reference normal, and on the adjacency relationships of the points (Step SB-6) .

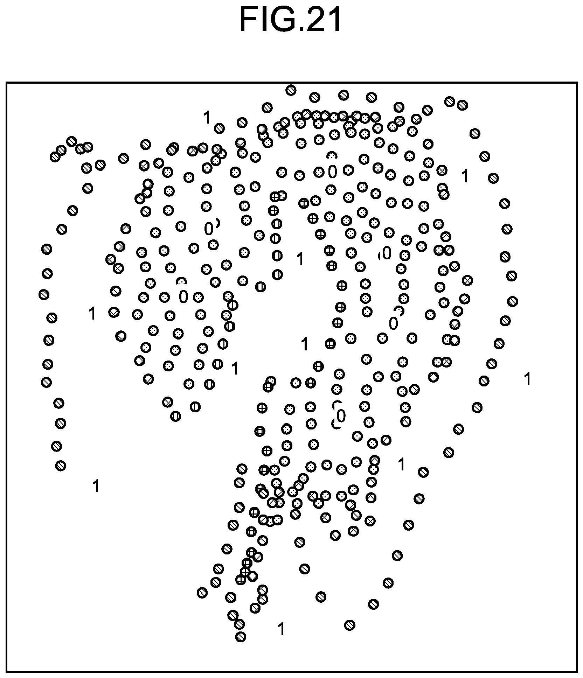

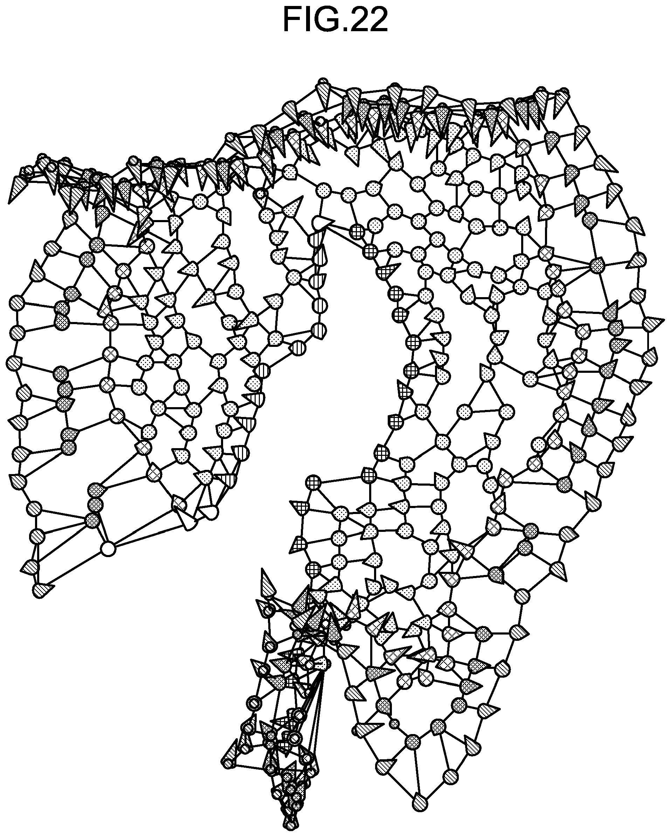

An example of an orientation correcting (unifying) process according to the embodiment will now be explained with reference to FIGS. 21 and 22. FIG. 21 is a schematic illustrating an example of labels for correcting the normals in the embodiment. FIG. 22 is a schematic illustrating an example of the orientation unification in the embodiment.

In the embodiment, in a process of correcting the orientations of the normals at the points of type 0, using the orientation of the normal at a predetermined point of type 0 as a reference for aligning (unifying) the orientations of the normals, the orientations of the normals at the adjacent points are corrected (unified) successively from the reference point.

In the embodiment, the orientations of the normals at the points of type 1, type 2, and type 3 are also corrected, in the same manner as for the points of type 0.

In the embodiment, for the points of type 4, the orientations of the normals are corrected only for those connected to the points of type 1, type 2, or type 3.

At this time, 0 meaning uncorrected is assigned to a point for which the orientation of the normal has not been corrected yet, and 1 meaning having been corrected or confirmed is assigned to a point for which the orientation of the normal has been corrected or confirmed, as illustrated in FIG. 21.

As illustrated in FIG. 21, for the points of type 4, 1 meaning having been corrected or confirmed is assigned only to those that are connected to the points of type 1, type 2, or type 3 via edges. 0 is assigned to the remaining points.

By performing this process until the orientations of all of the normals have been corrected or confirmed, as illustrated in FIG. 22, an initial shape with more accurate normals can be acquired.

Referring back to FIG. 13, the normal acquiring unit 102d selects a reference point from the points, weighs the normal at each of the points at the vertices adjacent to the reference point, based on the distance between the reference point and the adjacent vertex, using a Gaussian function, and performs smoothing of the normal at the reference point based on the weighing (Step SB-7).

The normals are smoothed in the embodiment so that the directions of the normals (normal vectors) are brought closer to the shape of mesentery.

In the embodiment, the membrane structure is estimated using normal vectors, but it is impossible to reconstruct a shape by an RBF interpolation unless the orientations of the normals are aligned to one direction, with respect to the orientation of a plane.

To address this issue, in the embodiment, the normal smoothing is performed by weighing the orientations of the normals at adjacent points, based on the distances between the reference point and the adjacent points, using a three-dimensional Gaussian function indicated as Equation 4 below.

[Equation 4]

$$f\left(x,\ y,\ z\right)\ =\ \frac{1}{\left(\sqrt{2\pi}\right)^3\sigma^3}\ \exp\left(-\ \frac{x^2\ +\ y^2\ +\ z^2}{2\sigma^2}\right) \tag{4}$$

The membrane structure estimating unit 102e then estimates the membrane structure by creating an isosurface based on the coordinates of the points and the normals at the points, using an RBF interpolation that is based on a radial basis function (Step SB-8).

The membrane structure estimating unit 102e then causes the input/output unit 112 to display the membrane structure, and determines whether a user has entered any instruction for adding a point and/or deleting the point via the input/output unit 112, for each of a predetermined number of labels (Step SB-9).

If the membrane structure estimating unit 102e determines that an instruction for adding points and/or deleting the points has been entered at Step SB-9 (Yes at Step SB-9), the process is shifted to Step SB-10.

Based on the instruction entered by the user at Step SB-9, the point acquiring unit 102b acquires a point group resultant of adding points to and/or deleting the points from the point group included in the initial shape in the medical image, for each of a predetermined number of labels (Step SB-10), and the process is shifted to Step SB-3.

If the membrane structure estimating unit 102e determines that no instruction for adding points and/or deleting the points has been entered at Step SB-9 (No at Step SB-9), the process is shifted to Step SB-11.

The membrane structure estimating unit 102e then causes the input/output unit 112 to display the normals, and determines whether a user has entered an instruction for changing the normals, adding normals, and/or deleting the normal at any point, via the input/output unit 112 (Step SB-11).

If the membrane structure estimating unit 102e determines that a change of the normal, an addition of a normal, and/or a deletion of the normal at a point has been entered at Step SB-11 (Yes at Step SB-11), the process is shifted to Step SB-12.

The normal acquiring unit 102d then changes the normal, adds a normal, and/or deletes the normal at the point included in the initial shape, based on the instruction entered by the user at Step SB-11 (Step SB-12), and the process is shifted to Step SB-8.

If the membrane structure estimating unit 102e determines that a change of the normal, an addition of a normal, and/or a deletion of the normal at a point has not been entered at Step SB-11 (No at Step SB-11), the process is shifted to Step SB-13.

The image reconstructing unit 102f creates a reconstruction image corresponding to a clipped section of the mesentery, based on the membrane structure estimated by the membrane structure estimating unit 102e (Step SB-13).

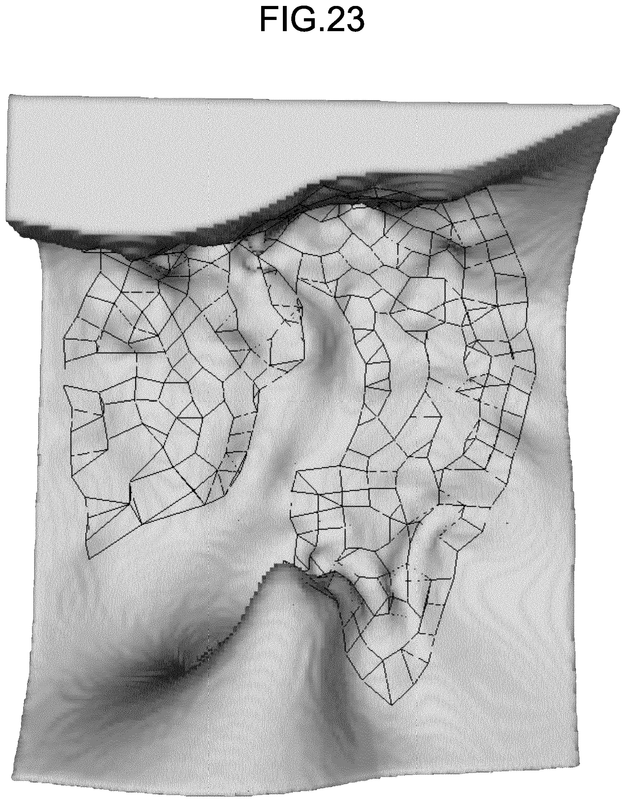

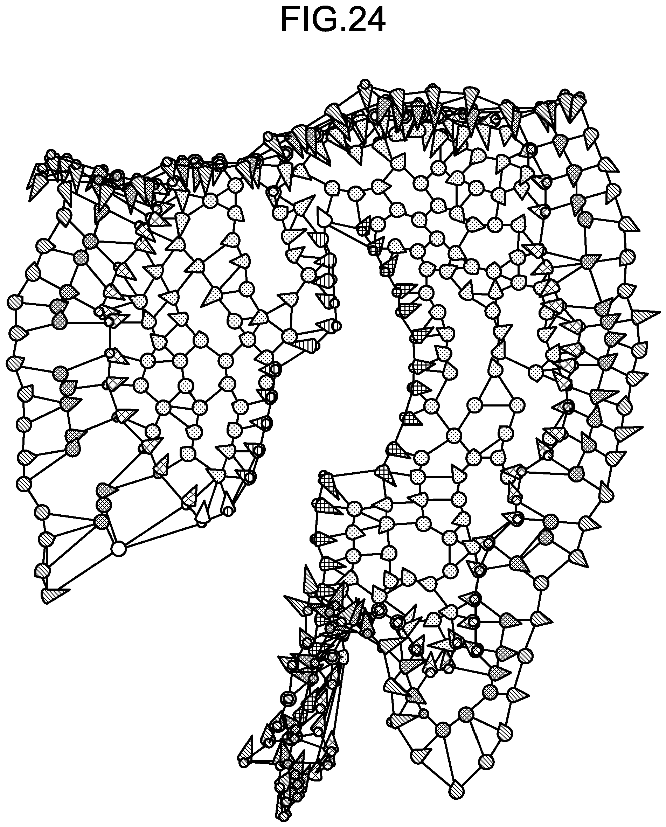

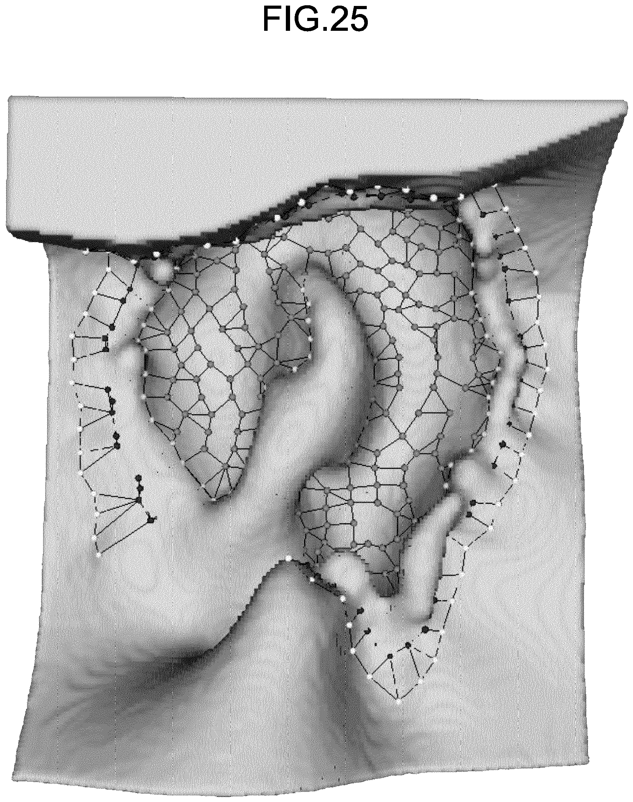

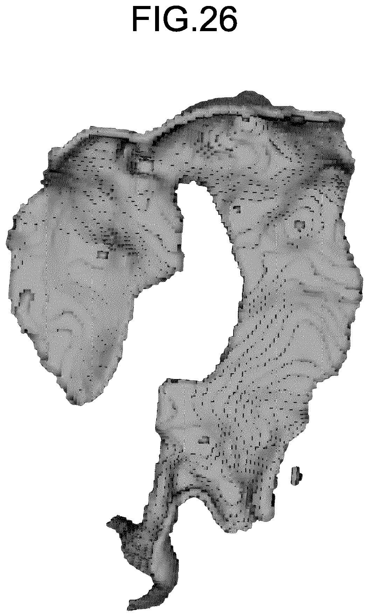

An example of an image clipping process according to the embodiment will now be explained with reference to FIGS. 23 to 26. FIG. 23 is a schematic illustrating an example of volume data before the clipping in the embodiment. FIG. 24 is a schematic illustrating an example in which the normals at the points of types 1 to 3 are directed to the normals at the points of type 4 in the embodiment. FIG. 25 is a schematic illustrating an example of volume data that is to be subjected to the clipping according to the embodiment. FIG. 26 is a schematic illustrating an example of volume data after the clipping according to the embodiment.

In the embodiment, a resynthesized image may be created by clipping only the mesentery volume data from the mesentery volume data including the estimated colon boundary, by an RBF interpolation.

In the embodiment, in order to clip only the mesentery volume data from the volume data illustrated in FIG. 23, the normal vectors of type 1, type 2, and type 3 representing the boundary around the mesentery shape are directed toward type 4, as illustrated in FIG. 24, and the structure is estimated by the RBF interpolation.

In this manner, in the embodiment, it is possible to reconstruct volume data representing having only the mesentery removed, as illustrated in FIG. 25.

In the embodiment, the volume data of the mesentery only is then clipped from the volume data illustrated in FIG. 23, based on the difference between the shape of mesentery in the volume data illustrated in FIG. 23 and that in volume data illustrated in FIG. 25, as illustrated in FIG. 26.

Referring back to FIG. 13, the image outputting unit 102g causes the input/output unit 112 to display the reconstruction image as simulation data (Step SB-14), and the process is ended.

Explanation of the example of the process performed by the structure estimating apparatus 100 according to the embodiment is now ended.


Other Embodiments


Some embodiments of the present invention are explained above, but in addition to the embodiments described above, various different embodiments of the present invention are still possible, within the scope of the technological idea described in the appended claims.

For example, explained above is an example in which a standalone structure estimating apparatus 100 performs the process, but the structure estimating apparatus 100 may perform the process in response to a request from a client terminal (a housing provided separately from the structure estimating apparatus 100), and return the result of the process to the client terminal.

The processes explained to be performed automatically in the embodiment may be performed entirely or partly manually, or the processes explained to be performed manually in the embodiment may be performed entirely or partly automatically using some known method.

In addition, the processing sequence, controlling sequence, specific names, information including parameters such as registration data or retrieval conditions used in each process, exemplary screens, and database configurations disclosed in the above literature or in the drawings may be modified in any way, except specified otherwise.

Furthermore, in relation to the structure estimating apparatus 100, the units included therein illustrated in the drawings are merely functional and conceptual representations, and do not need to be physically configured in the manner as illustrated in the drawings.

For example, the processing functions provided to each of the devices included in the structure estimating apparatus 100, the functions of the processes executed by the control unit 102, in particular, may be partly or entirely implemented by a central processing unit (CPU) and a computer program parsed and executed by the CPU, or by hardware using a wired logic. The computer program is stored in a non-volatile computer-readable recording medium including instructions programed to cause a computer to execute a method according to the present invention, which is described later, and is mechanically read by the structure estimating apparatus 100 as required. In other words, the storage unit 106 such as a ROM or a hard disk drive (HDD) stores therein a computer program for issuing instructions to the CPU and causing the CPU to execute various processes by cooperating with an operating system (OS). This computer program is executed by being loaded to the RAM, and implements the control unit by cooperating with the CPU.

The computer program may also be stored in an application program server that is connected to the structure estimating apparatus 100 over a network, and the entire or a part of the computer program may be downloaded as required.

The computer program according to the present invention may be stored in a computer-readable recording medium, or provided as a computer program product. The "recording medium" herein includes any "portable physical medium" such as a memory card, a universal serial bus (USB) memory, a Secure Digital (SD) card, a flexible disk, a magneto-optical disk, a ROM, an erasable programmable read-only memory (EPROM) (registered trademark), an electrically erasable and programmable read-only memory (EEPROM), a compact disc read-only memory (CD-ROM), a digital versatile disc (DVD), and a Blu-ray (registered trademark) disc.

The "computer program" is a method of data processing described in some language or description method, and the format thereof is not limited to a particular method such as a source code or a binary code. The "computer program" is not limited to a configuration including only one computer program, and also includes a computer program implemented as a plurality of modules or libraries in a distributed manner, or a computer program achieving its functions by cooperating with a separate computer program, a representative example of which is an operating system (OS). In each of the apparatuses explained in the embodiment, any known configuration and procedures may be used as the specific configuration and the procedure for reading the recording medium, and the installation procedure after reading the recording medium.

Various databases or the like stored in the storage unit 106 are a storage unit such as a memory device including a RAM and a ROM, a fixed disk device such as a hard disk drive, a flexible disk, and an optical disk, and store therein various computer programs, tables, databases, and files for web pages, used in executing various processes and providing a web site.

The structure estimating apparatus 100 may be provided as a known desktop or laptop personal computer, a mobile terminal device such as a mobile phone, a smartphone, a personal handy phone system (PHS), and a personal digital assistant (PDA), or an information process terminal such as a workstation, and may be configured as such an information process terminal that is connected with a peripheral device. The structure estimating apparatus 100 may be implemented as a software implementation (including a computer program and data) for causing such an information process terminal to implement the method according to the present invention.

The configuration in which the apparatus is distributed or integrated is not limited to those illustrated, and the entire or a part of the configuration may be functionally or physically distributed or integrated to any units, depending on various additions or functional loads. In other words, the embodiment described above may be combined in any way, or the embodiment may be implemented selectively.

Industrial Applicability

As explained above in detail, according to the present invention, it is possible to provide a structure estimating apparatus, a structure estimating method, and a computer program capable of visualizing and modelling a membrane structure in which, in principle, it is impossible to achieve an appropriate contrast using a medical image that is unique to a patient. Therefore, the present invention is extremely useful in various fields, particularly in medicines, medical educations, and biological studies, for example.

Reference Signs List

    100 structure estimating apparatus
    102 control unit
    102a image acquiring unit
    102b point acquiring unit
    102c initial shape creating unit

102d normal acquiring unit
102e membrane structure estimating unit
102f image reconstructing unit
102g image outputting unit
106 storage unit
106a image database
112 input/output unit

## Claims

1. A structure estimating apparatus comprising:

   a point acquiring unit that acquires a plurality of points that are present in a target membrane in a medical image;
   an initial shape creating unit that creates an initial shape of a polyhedron including the points;
   a normal acquiring unit that acquires a normal at each of the points; and
   a membrane structure estimating unit that estimates a membrane structure by creating an isosurface using a radial basis function based on coordinates of and the normals at the points.

2. The structure estimating apparatus according to claim 1, wherein the initial shape creating unit creates the initial shape of the polyhedron including the points based on a predetermined number of labels corresponding to coordinates.

3. The structure estimating apparatus according to claim 2, wherein the initial shape creating unit creates the initial shape of the polyhedron including the points based on a relation of connection between the points according to the labels, distances between the points, and a maximum number of edges of the polyhedron having such points as end points.

4. The structure estimating apparatus according to any one of claims 1 to 3, wherein the normal acquiring unit selects a reference point from the points, selects a predetermined number of points near the reference point, calculates normals of a predetermined number of triangles formed by the reference point and the points near the reference point, calculates an average normal that is an average of the normals of the predetermined number of triangles, and acquires the average normal as a normal at the reference point.

5. The structure estimating apparatus according to claim 2, wherein the normal acquiring unit also determines a reference normal from normals at points that are assigned with a same label, and corrects an orientation of the normals at the points that are assigned with the same label, based on a direction of the reference normal and adjacency relationships of the points.

6. The structure estimating apparatus according to any one of claims 1 to 3, wherein the normal acquiring unit also selects a reference point from the points, weighs the normal at each of the points at vertices adjacent to the reference point, based on the distance between the reference point and the adjacent vertex, using a Gaussian function, and performs smoothing of the normal at the reference point based on the weighing.

7. The structure estimating apparatus according to any one of claims 1 to 3, wherein
   the point acquiring unit also acquires a predetermined number of supplementary points between two points that are end points of an edge of the polyhedron in the medical image, and
   the normal acquiring unit acquires the normal at each of the points based on the positions of the supplementary points.

8. The structure estimating apparatus according to any one of claims 1 to 3, wherein the normal acquiring unit acquires the normal at a selected point that is a point selected from the points.

9. The structure estimating apparatus according to claim 8, wherein the normal acquiring unit causes the initial shape to be displayed, prompts a user to select the selected point from the points, and acquires the normal at the selected point.

10. The structure estimating apparatus according to claim 8 or 9, wherein the normal acquiring unit acquires the normal at the selected point based on a plane that includes the selected point by which the polyhedron is formed.

11. The structure estimating apparatus according to any one of claims 1 to 3, wherein the normal acquiring unit causes the initial shape to be displayed, prompts a user to make any one or both of an addition of points and a deletion from the points, and acquires the normals at each of the points.

12. The structure estimating apparatus according to claim 2, wherein the point acquiring unit acquires the points that are present in the target membrane, for each of a predetermined number of labels, in the medical image.

13. The structure estimating apparatus according to any one of claims 1 to 11, wherein the point acquiring unit acquires the points that are present in the target membrane, by extracting points in a structure other than the membrane by region growing in the medical image.

14. The structure estimating apparatus according to any one of claims 1 to 8, wherein the point acquiring unit acquires the points by displaying the medical image and prompting a user to enter the points that are present in the target membrane on the medical image.

15. The structure estimating apparatus according to any one of claims 1 to 8, wherein the point acquiring unit acquires the points by displaying the medical image, prompting a user to enter a line delineating the target membrane on the medical image, and identifying the points on the line.

16. The structure estimating apparatus according to any one of claims 1 to 15, wherein the membrane structure estimating unit estimates the membrane structure by creating the isosurface based on coordinates of and the normals at the points, using an RBF interpolation that is based on the radial basis function.

17. The structure estimating apparatus according to any one of claims 1 to 8, wherein the membrane structure estimating unit also causes the membrane structure to be displayed and prompts a user to make any one or both of an addition of points and a deletion from the points.

18. The structure estimating apparatus according to any one of claims 1 to 8, wherein the membrane structure estimating unit also causes the membrane structure to be displayed and prompts a user to make any one, some or all of a change of the normals, an addition of normals, and a deletion from the normals.

19. The structure estimating apparatus according to any one of claims 1 to 18, further comprising:

an image reconstructing unit that creates a reconstruction image that is a reconstruction of the medical image, based on the membrane structure estimated by the membrane structure estimating unit; and
an image outputting unit that outputs the reconstruction image.

20. The structure estimating apparatus according to claim 19, wherein the image reconstructing unit creates a reconstruction image corresponding to a clipped section of the membrane based on the membrane structure estimated by the membrane structure estimating unit.

21. The structure estimating apparatus according to claim 19, wherein the image reconstructing unit also creates a reconstruction image in which a structure other than the membrane is visualized based on the medical image, by volume rendering.

22. The structure estimating apparatus according to any one of claims 19 to 21, wherein
the image reconstructing unit also acquires a value indicating a reliability of the membrane structure estimated by the membrane structure estimating unit, and
the image outputting unit also causes the value indicating the reliability of the membrane structure to be displayed.

23. The structure estimating apparatus according to any one of claims 1 to 22, wherein the medical image is a CT image or an MRI image in which contrast of the membrane structure is not visually recognizable.

24. A structure estimating method executed by a structure estimating apparatus comprising:

a point acquiring step of acquiring a plurality of points that are present in a target membrane in a medical image;
an initial shape creating step of creating an initial shape of a polyhedron including the points;
a normal acquiring step of acquiring a normal at each of the points; and

a membrane structure estimating step of estimating a membrane structure by creating an isosurface using a radial basis function based on coordinates of and the normals at the points.

25. A computer program product having a non-transitory tangible computer readable medium including programmed instructions for causing, when executed by a computer,
the computer to perform a structure estimating method comprising:

a point acquiring step of acquiring a plurality of points that are present in a target membrane in a medical image;
an initial shape creating step of creating an initial shape of a polyhedron including the points;
a normal acquiring step of acquiring a normal at each of the points; and
a membrane structure estimating step of estimating a membrane structure by creating an isosurface using a radial basis function based on coordinates of and the normals at the points.

# FIG.1

# FIG.2

# FIG.3

# FIG.4

M

# FIG.5

# FIG.6

# FIG.7

# FIG.8

# FIG.9

# FIG.10

# FIG.11

# FIG.12

# FIG.13

START

ACQUIRE MEDICAL IMAGE — SB-1

ACQUIRE POINT — SB-2

CREATE INITIAL SHAPE — SB-3

ACQUIRE SUPPLEMENTARY POINT — SB-4

ACQUIRE NORMAL — SB-5

CORRECT ORIENTATION — SB-6

PERFORM NORMAL SMOOTHING — SB-7

ESTIMATE MEMBRANE STRUCTURE — SB-8

ADD/DELETE POINT? — SB-9

YES

SB-10
ADD/DELETE

NO

CHANGE/ADD/ DELETE NORMAL? — SB-11

YES

SB-12
CHANGE/ADD/ DELETE

NO

CREATE RECONSTRUCTION IMAGE — SB-13

DISPLAY RECONSTRUCTION IMAGE — SB-14

END

# FIG.14

| | type0 : COLON |
| type1 : MEMBRANE NEAREST TO COLON |
| type2 : RIGHT SIDE OF AORTA |
| type3 : LEFT SIDE OF AORTA |
| type4 : POINTS ON MEMBRANE |
| type5 : MIDPOINTS BETWEEN TYPE 0 AND TYPE 1 |

# FIG.15

| TYPE OF START POINT | TYPE OF END POINT | THRESHOLD | MAXIMUM NUMBER OF EDGES |
|---|---|---|---|
| 0 | 0 | 2.5 | 2 |
| 1 | 1 | 2.5 | 2 |
| 2 | 2 | 2.5 | 2 |
| 3 | 3 | 2.5 | 2 |
| 5 | 5 | 2.0 | 2 |
| 0 | 5 | 2.0 | 1 |
| 1 | 5 | 2.0 | 1 |
| 1 | 4 | 2.0 | 1 |
| 2 | 4 | 2.0 | 1 |
| 3 | 4 | 2.0 | 1 |
| 4 | 123 | 3.5 | 3 |

# FIG.16

# FIG.17

# FIG.18

EDGE

POLYLINE EDGE

# FIG.19

1

3

2

● REFERENCE POINT

◉ NEARBY POINT

# FIG.20

# FIG.21

# FIG.22

# FIG.23

# FIG.24

# FIG.25

# FIG.26

## INTERNATIONAL SEARCH REPORT

| International application No. |
|---|
| PCT/JP2016/076246 |

**A. CLASSIFICATION OF SUBJECT MATTER**
*A61B6/03*(2006.01)i, *A61B5/055*(2006.01)i, *G06T1/00*(2006.01)n, *G06T17/30* (2006.01)n

According to International Patent Classification (IPC) or to both national classification and IPC

**B. FIELDS SEARCHED**

Minimum documentation searched (classification system followed by classification symbols)
A61B6/03, A61B5/055, G06T1/00, G06T17/30

Documentation searched other than minimum documentation to the extent that such documents are included in the fields searched
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2009-247505 A (Fujifilm Corp.), 29 October 2009 (29.10.2009), entire text; all drawings & EP 2269512 A1 | 1-25 |
| A | JP 2010-178876 A (Toshiba Corp.), 19 August 2010 (19.08.2010), entire text; all drawings & US 2010/0195887 A1 | 1-25 |

☐ Further documents are listed in the continuation of Box C.     ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 15 November 2016 (15.11.16) | 29 November 2016 (29.11.16) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010131047 A